# FASCICULE DE BREVET EUROPEEN

(11) **EP 1 696 913 B1**
(45) Date de publication et mention de la délivrance du brevet: **04.03.2009**
(21) Numéro de dépôt: 04816422.2
(22) Date de dépôt: 17.12.2004
(51) Int. Cl.: A61K 31/4375, A61P 35/00, A61K 9/08, A61K 47/12

(54) **COMPOSITION PHARMACEUTIQUE DE VINFLUNINE DESTINÉE À L'ADMINISTRATION PARENTÉRALE, PROCÉDÉ DE PRÉPARATION ET UTILISATION**
PHARMAZEUTISCHE ZUSAMMENSETZUNG, VINFLUNIN ENTHALTEND, ZUR PARENTERALEN VERABREICHUNG, DEREN HERSTELLUNGSVERFAHREN UND ANWENDUNG
PHARMACEUTICAL COMPOSITION OF VINFLUNINE FOR PARENTERAL ADMINISTRATION, PREPARATION METHOD THEREOF AND USE OF SAME

(30) Priorité: 23.12.2003 FR 0315312
(43) Date de publication de la demande: 06.09.2006
(73) Titulaire: PIERRE FABRE MEDICAMENT, 92100 Boulogne-Billancourt (FR)
(72) Inventeur: LEVERD, Elie, F-81100 Castres (FR); BOUGARET, Joël, F-31460 Francarville (FR); IBARRA, Marie-Dominique, F-11400 Souilhanels (FR)
(74) Mandataire: Ahner, Francis
(86) Numéro de dépôt international: PCT/FR2004/003287
(87) Numéro de publication internationale: WO 2005/070425

(56) Documents cités:
- EP-A- 0 298 192
- US-A- 4 619 935
- US-A- 4 923 876
- HILL BRIDGET T: "Vinflunine, a second generation novel Vinca alkaloid with a distinctive pharmacological profile, now in clinical development and prospects for future mitotic blockers" septembre 2001 (2001-09), CURRENT PHARMACEUTICAL DESIGN, VOL. 7, NR. 13, PAGE(S) 1199-1212 , XP002297712 ISSN: 1381-6128 figure 1 page 1208, colonne de gauche, ligne 36-42 page 1208, colonne de droite, ligne 31-39 tableaux 1,3
- HILL B T ET AL: "Superior in vivo experimental antitumour activity of vinflunine, relative to vinorelbine, in a panel of human tumour xenografts." EUROPEAN JOURNAL OF CANCER (OXFORD, ENGLAND : 1990) MAR 1999, vol. 35, no. 3, mars 1999 (1999-03), pages 512-520, XP002297710 ISSN: 0959-8049
- KRUCZYNSKI A ET AL: "Preclinical in vivo antitumor activity of vinflunine, a novel fluorinated Vinca alkaloid" CANCER CHEMOTHERAPY AND PHARMACOLOGY 1998 GERMANY, vol. 41, no. 6, 1998, pages 437-447, XP002297711 ISSN: 0344-5704

## Description

La présente invention concerne une composition pharmaceutique pour l'administration parentérale de vinflunine.

L'étude des propriétés anti-néoplasiques des alcaloïdes de Vinca Roséa (famille des Apocynacées) a déjà permis de mettre en évidence les activités intéressantes de composés à structure indolique comme la vincristine, la vinblastine ou leurs dérivés comme la vinflunine : 20',20'-difluoro-3',4'-dihydrovinorelbine de formule (a) suivante : 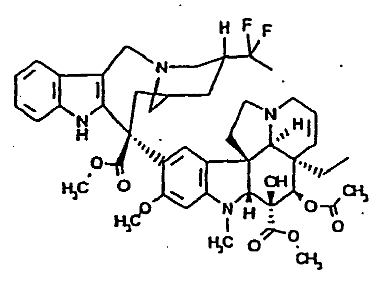 décrit dans le brevet EP 0 710 240.

Mais la mise au point des formulations injectables de ces principes actifs s'est toujours heurtée à des problèmes liés à leur stabilité en solution aqueuse.

Durant de très nombreuses années, seule la forme lyophilisée a été commercialisée. Nécessitant une reconstitution extemporanée avec le contenu d'une ampoule solvant avant administration, le lyophilisat présentait des inconvénients majeurs liés aux dangers engendrés par sa manipulation :
- Risque d'une reconstitution effectuée avec maladresse durant laquelle de fines gouttelettes de produit sont générées et peuvent contaminer le personnel soignant ou les locaux;
- Utilisation d'une mauvaise quantité de solvant ou d'une quantité inappropriée de principe actif si la spécialité pharmaceutique est présentée en différents flacons correspondant à différents dosages unitaires.

Ce dernier point est particulièrement important. Il illustre les possibilités potentielles d'une dose non thérapeutique administrée au patient ou d'une exposition de celui-ci à un surdosage accidentel.

Le brevet US 4,619,935 a laissé entrevoir la possibilité de formuler des solutions injectables prêtes à l'emploi pour les alcaloïdes du Vinca.

Cependant, les formules mises en oeuvre sont complexes. Elles comprennent, en plus du principe actif :
- un sucre ou un polyol dérivé d'un sucre, comme le mannitol,
- un tampon acétate, pour maintenir le pH de la solution dans l'intervalle 3,0 - 5,0, et plus particulièrement dans l'intervalle 4,4 - 4,8. Sa molarité est comprise entre 0,02 et 0,0005 M, préférentiellement 0,01 et 0,002 M,
- des conservateurs anti-microbiens.

Il est à remarquer que malgré l'effet stabilisant attribué au tampon acétate, qui permet de prévenir toute dégradation due à un changement de pH causé par la décomposition des alcaloïdes, la formulation objet de l'invention ne jouit que d'une stabilité de 1 an à 5°C.

La complexité des formules brevetées est allée croissante : le brevet FR 2 653 998 décrit une composition pharmaceutique à usage parentéral, contenant un alcaloïde de type bis-indole tel que la vincristine, la vinblastine ou la 5'-nor-anhydrovinblastine. Elle est caractérisée en ce qu'elle comprend en solution aqueuse un complexe de zinc d'un sel d'alcaloïde de type bis-indole, un gluconate de métal bivalent et un agent conservateur dissous dans un alcool mono ou polyhydrique.

La stabilité indiquée pour ces compositions est d'au moins 24 mois quand elles sont conservées au réfrigérateur.

Le brevet européen EP 0 298 192 met en avant l'effet favorable des sels de l'acide éthylènediaminetetracétique, en particulier le sel de sodium, sur la stabilité des solutions aqueuses d'alcaloïdes dimères du Vinca. Ces solutions aqueuses sont tamponnées avec un tampon acétate afin de maintenir le pH entre les valeurs 3,0 à 5,5, préférentiellement 4,0 à 5,0.

Dans ces conditions, en regard des spécifications retenues (teneur en alcaloïde comprise entre 90 % et 110 % de la teneur théorique), la solution reste stable 30 mois à une température de 2 à 8°C.

Le brevet canadien 2,001,643, relatif à une solution injectable de vincristine, met aussi l'accent sur la nécessité de mettre en oeuvre un tampon acide acétique / acétate de sodium pour maintenir le pH de la solution entre les valeurs 3,5 et 5,5, plus particulièrement entre 4,0 et 4,5. La formulation décrite dans l'invention est stable 18 mois à 5°C, et peut-être même 24 mois à 5°C.

Le ditartrate de vinflunine ou 20',20'-difluoro-3',4'-dihydrovinorelbine L - (+) tartrate est une poudre blanche qui doit être conservée à une température négative, inférieure à - 15°C, sous une atmosphère de gaz inerte comme l'azote ou l'argon.

De façon tout à fait inattendue, il a été trouvé que le ditartrate de vinflunine était beaucoup plus stable une fois dissous dans l'eau que sous forme pulvérulente.

La solution aqueuse injectable est en effet conservée à une température positive, comprise entre + 2°C et + 8°C. Ceci est totalement surprenant car il est bien connu que les réactions chimiques de dégradation surviennent plus facilement en milieu liquide qu'à l'état solide.

La présente invention concerne donc une composition pharmaceutique de vinflunine, caractérisée en ce qu'elle se présente sous la forme d'une solution aqueuse stable et stérile d'un sel hydrosoluble de vinflunine à un pH compris entre 3 et 4.

L'objet de l'invention repose sur l'extraordinaire simplicité de la formulation qui contraste avec les compositions décrites dans les brevets rappelés initialement.

Avantageusement, le sel de vinflunine est le ditartrate de vinflunine.

Avantageusement, la composition pharmaceutique selon la présente invention se présente sous forme d'une solution aqueuse stable, stérile et apyrogène, prête à l'emploi, injectable.

Avantageusement, la composition selon la présente invention ne contient aucun conservateur.

Dans un premier mode de réalisation de la présente invention, la composition pharmaceutique selon la présente invention se présente sous forme d'une solution aqueuse simple de ditartrate de vinflunine, sans ajout de solution tampon. La composition est ainsi constituée par le ditartrate de vinflunine et de l'eau pour préparation injectable. Avantageusement, le pH de cette solution est égal à 3,5.

Dans un deuxième mode de réalisation de la présente invention, la composition pharmaceutique selon la présente invention comprend un système tampon pH afin de maintenir le pH entre 3 et 4. De façon encore plus avantageuse, la composition pharmaceutique selon la présente invention est constituée par le ditartrate de vinflunine, de l'eau pour préparation injectable et un tampon pH afin de maintenir le pH entre 3 et 4. De façon avantageuse la molarité du système tampon pH est comprise entre 0,002 M et 0,2 M.

Avantageusement, le système tampon est constitué par un tampon acide acétique / acétate de sodium ou un tampon acide citrique/ citrate de sodium.

De façon avantageuse, le pH est obtenu avec des solutions tampons acide acétique / acétate de sodium ou acide citrique / citrate de sodium de molarités comprises entre 0,05 M et 0,2 M.

De façon encore plus avantageuse, le tampon pH est constitué par le tampon acide acétique / acétate de sodium et le pH de la composition est alors de 3,5 ou le tampon pH est constitué par le tampon acide citrique / citrate de sodium et le pH de la composition est alors de 4.

Avantageusement, la composition selon la présente invention contient du ditartrate de vinflunine en une concentration en vinflunine de base comprise entre 1 et 50 mg/ml, avantageusement comprise entre 25 et 30 mg/ml, en particulier 25 mg/ml ou 30 mg/ml. Cette concentration est donc exprimée en vinflunine base. La quantité administrée est fonction de la surface corporelle des patients.

Dans un mode de réalisation avantageux, la composition selon la présente invention répond à l'une des formules suivantes : 68,35 mg de vinflunine ditartrate qsp 2ml en eau ou 136,70 mg de vinflunine ditartrate qsp 4 ml d'eau ou 341,75 mg de vinflunine ditartrate qsp 10 ml d'eau, la quantité de vinflunine ditartrate correspondant respectivement dans chacune des formules à 50 mg de vinflunine de base, 100 mg de vinflunine de base et 250 mg de vinflunine de base. Ces données sont rassemblées dans le tableau 1 ci-dessous.

**Tableau 1 : exemples de compositions unitaires de la solution aqueuse**

| **NOM DES COMPOSANTS** | **DOSAGES UNITAIRES VINFLUNINE** | | |
|---|---|---|---|
| Vinflunine ditartrate | 68,35 mg | 136,70 mg | 341,75 mg |
| correspondant à Vinflunine base | 50,00 mg | 100,00 mg | 250,00 mg |
| | | | |
| Eau pour préparations injectables | qsp 2 ml | qsp 4 ml | qsp 10 ml |

Le tableau 1 ci-dessus montre la possibilité de préparer en flacons 3 dosages unitaires de vinflunine résultant de la répartition en différents volumes d'une même solution aqueuse de ditartrate de vinflunine ayant une concentration de 25 mg/ml exprimée en vinflunine base.

Dans un autre mode de réalisation de l'invention, la composition selon la présente invention reste stable pendant au moins 36 mois à 5°C ± 3°C.

Dans un mode de réalisation particulier de l'invention, la composition pharmaceutique selon la présente invention est administrée par perfusion, en voie intra-veineuse, après sa dissolution dans des solutions de perfusion telles que les solutions de chlorure de sodium à 0,9 % ou de glucose à 5 %.

La présente invention concerne donc également la composition pharmaceutique selon la présente invention pour son utilisation comme médicament, en particulier pour traiter le cancer, avantageusement pour une administration parentérale, de façon avantageuse par voie intra-veineuse par perfusion, de façon encore plus avantageuse, lors de chimiothérapie en tant qu'antinéoplasique et antitumoral.

La présente invention concerne également l'utilisation d'une composition selon la présente invention pour la fabrication d'un médicament destiné à l'administration parentérale, avantageusement par voie intra-veineuse par perfusion, de façon avantageuse, destiné au traitement du cancer.

L'administration parentérale notamment par voie intra-veineuse, d'une composition pharmaceutique de vinflunine selon la présente invention permet de traiter les cancers sensibles à l'action de la vinflunine.

La présente invention concerne également un procédé de préparation d'une composition selon la présente invention comprenant les étapes successives suivantes :
- (a) dissolution du sel de vinflunine dans l'eau pour préparations injectables
- (b) addition éventuelle d'un tampon pH
- (c) stérilisation par filtration de la solution vrac.

Dans un mode de réalisation particulier de l'invention, le procédé selon la présente invention comprend l'étape supplémentaire (d) de répartition aseptique, sous atmosphère d'azote, de la composition stérile obtenue à l'étape (c) dans un conditionnement. Avantageusement, ce conditionnement est choisi parmi les ampoules de verre, préférentiellement type I ambré ou incolore, les flacons de verre préférentiellement type I ambré ou incolore munis d'un bouchon en élastomère et d'une cape aluminium sertie ou tout système prêt à l'emploi compatible comme par exemple une seringue pré-remplie.

La présente invention concerne donc également un récipient de conditionnement contenant la composition selon la présente invention.

Ce récipient de conditionnement peut être choisi parmi les ampoules de verre préférentiellement type I ambré ou incolore, les flacons de verre préférentiellement type I ambré ou incolore munis d'un bouchon en élastomère et d'une cape aluminium sertie ou tout système prêt à l'emploi compatible comme par exemple une seringue pré-remplie.

Les exemples suivants sont donnés à titre indicatif non limitatif.

### Exemple 1 : Comparaison de la stabilité entre le ditartrate de vinflunine sous forme pulvérulente et le ditartrate de vinflunine en solution aqueuse (composition selon la présente invention).

Le tableau 2 ci-dessous montre les résultats de stabilité obtenus pour un lot de ditartrate de vinflunine lyophilisé pulvérulent (lot 503) et un lot de solution aqueuse à 25 mg / ml de vinflunine base (lot SB0222) fabriqué avec ce même lot de ditartrate de vinflunine, après 3 mois et 6 mois de conservation à 25°C. La stabilité est suivie à travers l'évolution du total des impuretés présentes, apparentées à la vinflunine.

**Tableau 2 : résultats de stabilité ditartrate de vinfluninelsolution aqueuse**

| | **Ditartrate de vinflunine (lot 503)** | **Solution aqueuse à 25 mg / ml** |
|---|---|---|
| | **(% d'impureté par** | **(lot SB0222)** |
| | **rapport à 100 % de principe actif)** | **(% d'impureté par rapport à 100 % de principe actif)** |
| **t₀** | 1,17 | 1,23 |
| **t_{3 mois}** | 2,75 | 1,45 |
| **t_{6 mois}** | 3,48 | 2,00 |

Après 6 mois de conservation à 25°C, le total des impuretés apparentées à la vinflunine est augmenté de :
- 62 % dans la solution aqueuse de ditartrate de vinflunine,
- 197 % pour le ditartrate de vinflunine pulvérulent

### Exemple 2 : Etude de stabilité en fonction du pH des compositions selon la présente invention

Des études de stabilité ont été conduites sur des solutions aqueuses de ditartrate de vinflunine, dans un domaine de pH compris entre 2,5 et 5,0, et plus particulièrement entre 3,0 et 4,0. Le pH était obtenu avec des solutions tampons acide acétique / acétate de sodium ou acide citrique / citrate de sodium 0,2 molaires.

Les formules centésimales mises en oeuvre sont présentées dans le tableau 3 ci-après. Elles correspondent à une concentration en vinflunine de base de 30 mg / ml.

**Tableau 3 : formules des solutions aqueuses tamponnées**

| | **COMPOSITIONS** | | |
|---|---|---|---|
| | **BS1332** | **BS1330** | **BS1327** |
| | **(pH=3,5)** | **(pH=3,5)** | **(pH = 4,0)** |
| **Vinflunine ditartrate** | 4,101 g | 4,101 g | 4,101 g |
| **correspondant à Vinflunine base** | 3 g | 3 g | 3 g |
| **Acide acétique glacial** | 1,185 g | | |
| **Acétate de sodium** | 0,100 g | | |
| **Acide citrique monohydraté** | | 2,885 g | 2,460 g |
| **Citrate de sodium dihydraté** | | 1,903 g | 2,497 g |
| **Eau pour préparations injectables** | qsp 100 ml | qsp 100 ml | qsp 100 ml |

Les résultats ont été comparés à ceux concernant une solution aqueuse simple de ditartrate de vinflunine, sans ajout de solution tampon, conservée dans les mêmes conditions. Le pH de cette solution est égal à 3,5.

Les composition et références des solutions testées sont rassemblées dans le tableau4 ci-dessous.

**Tableau 4 : composition et référence des solutions testées**

| **Composition** | **Référence Formule** |
|---|---|
| Solution à pH = 2,5 (tampon citrate) | BS 1325 |
| Solution à pH = 3 (tampon citrate) | BS 1326 |
| Solution à pH = 3,5 (tampon citrate) | BS 1330 |
| Solution à pH = 4 (tampon citrate) | BS 1327 |
| Solution à pH = 5 (tampon citrate) | BS 1328 |
| Solution à pH = 3,5 (tampon acétate) | BS 1332 |
| Solution aqueuse non tamponnée | BS 1331 |

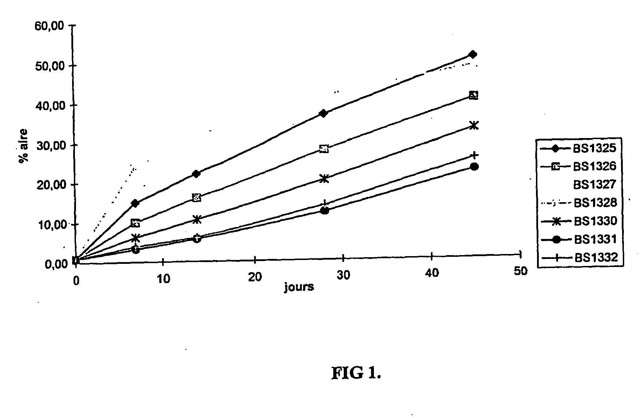

La Figure 1 représente l'évolution déterminée par HPLC de la teneur en impureté totales apparentées à la vinflunine en fonction du temps, dans des conditions stressantes (45 jours à 60°C), pour chaque formule indiquée dans le tableau 4.

Ils sont complétés par les résultats indiqués dans le tableau 5 ci-après de l'évolution de la couleur des solutions pendant 7 jours à 60°C.

Le suivi de l'absorbance de ces solutions, dans l'ultra-violet, à 410 nm, est révélateur de l'apparition de dérivés d'oxydation de la vinflunine non chromatographiés en HPLC.

**Tableau 5 : évolution de l'absorbance**

| **LOTS** | **Absorbance à 410 nm** | |
|---|---|---|
| | **t**₀ | **t**_{7 **jours**} |
| BS 1325 | | |
| pH = 2,5 | 0,021 | 0,645 |
| Tampon citrate : 0,2 M | | |
| BS 1326 | | |
| pH = 3,0 | 0,020 | 0,520 |
| Tampon citrate : 0,2 M | | |
| BS1330 | | |
| pH = 3,5 | 0,020 | 0,354 |
| Tampon citrate : 0,2 M | | |
| BS1327 | | |
| pH = 4,0 | 0,023 | 0,346 |
| Tampon citrate : 0,2 M | | |
| BS1328 | | |
| pH = 5,0 | 0,020 | 0,896 |
| Tampon citrate : 0,2 M | | |
| BS1332 | | |
| pH = 3,5 | 0,021 | 0,226 |
| Tampon acétate : 0,2 M | | |
| BS1331 | | |
| pH = 3,5 | 0,019 | 0,171 |
| Sans tampon | | |

Seule la solution non tamponnée, pH = 3,5, présente une absorbance inférieure à 0,200 après 7 jours à 60°C.

Les résultats indiquent que la stabilité de la vinflunine est meilleure pour une valeur de pH comprise entre 3,0 et 4,0 mais est dépendante de la nature des ions composant le tampon. A pH = 3,5, le tampon acide acétique / acétate de sodium assure une meilleure stabilité que le tampon acide citrique / citrate de sodium. Pour ce dernier tampon, les résultats sont meilleurs à pH = 4.

De façon tout à fait surprenante, il s'avère que la stabilité de la solution aqueuse de ditartrate de vinflunine, à son pH instantané de 3,5, est meilleure que la stabilité des solutions aqueuses de ditartrate de vinflunine tamponnées à pH = 3,5.

Ces bons résultats sont confirmés par les résultats à long terme rassemblés dans le tableau 6 ci-après qui indiquent que la composition pharmaceutique aqueuse injectable de vinflunine selon la présente invention peut être conservée au moins 36 mois à 5°C ± 3°C sans subir de dégradation importante.

**Tableau 6: résultats de stabilité de la composition pharmaceutique aqueuse selon la présente invention**

| | **t₀** | **t_{3 mois}** | **t_{6 mois}** | **t_{12 mois}** | **t_{24 mois}** | **t_{36 mois}** |
|---|---|---|---|---|---|---|
| **Lot CLP004** | | | | | | |
| **Teneur en vinflunine en mg /ml (théorie = 30,0)** | 30,8 | 30,4 | 30,4 | 30,4 | 30,3 | 30,2 |

## Revendications

1. Composition pharmaceutique de vinflunine, **caractérisée en ce qu'**elle se présente sous la forme d'une solution aqueuse stable et stérile d'un sel hydrosoluble de vinflunine à un pH compris entre 3 et 4 et qu'elle ne contient aucun conservateur.

2. Composition selon la revendication 1, **caractérisée en ce que** le sel de vinflunine est le ditartrate de vinflunine.

3. Composition selon la revendication 2, **caractérisée en ce que** la composition est constituée par le ditartrate de vinflunine et de l'eau pour préparation injectable.

4. Composition selon la revendication 1 ou 2, **caractérisée en ce qu'**elle comprend un système tampon pH afin de maintenir le pH entre 3 et 4.

5. Composition selon la revendication 4, **caractérisé en ce que** la molarité du système tampon pH est comprise entre 0,002 M et 0,2 M.

6. Composition selon l'une quelconque des revendications 4 ou 5, **caractérisée en ce que** le système tampon pH est constitué par un tampon acide acétique / acétate de sodium ou un tampon acide citrique/ citrate de sodium

7. Composition selon l'une quelconque des revendications 2 à 6, **caractérisée en ce que** la composition contient du ditartrate de vinflunine en une concentration en vinflunine de base comprise entre 1 et 50 mg/ml, avantageusement comprise entre 25 et 30 mg/ml, en particulier 25 mg/ml.

8. Composition selon l'une quelconque des revendications 2 à 7, **caractérisée en ce qu'**elle répond à l'une des formules suivantes : 68,35 mg de vinflunine ditartrate qsp 2ml en eau ou 136,70 mg de vinflunine ditartrate qsp 4 ml d'eau ou 341,75 mg de vinflunine ditartrate qsp 10 ml d'eau, la vinflunine ditartrate correspondant respectivement à 50 mg de vinflunine de base, 100 mg de vinflunine de base et 250 mg de vinflunine de base.

9. Composition selon l'une quelconque des revendications précédentes **caractérisé en ce qu'**elle reste stable pendant au moins 36 mois à 5°C ± 3°C.

10. Utilisation d'une composition selon l'une quelconque des revendications 1 à 9 pour la fabrication d'un médicament destiné à l'administration parentérale, avantageusement par voie intra-veineuse par perfusion.

11. Utilisation selon la revendication 10 **caractérisé en ce que** le médicament est destiné au traitement du cancer.

12. Procédé de préparation d'une composition selon l'une quelconque des revendications 1 à 9 comprenant les étapes successives suivantes :
- (a) dissolution du sel de vinflunine dans l'eau pour préparations injectables
- (b) addition éventuelle d'un tampon pH
- (c) stérilisation par filtration de la solution vrac.
- (d) répartition aseptique, sous atmosphère d'azote, de la composition stérile obtenue à l'étape (c) dans le conditionnement choisi, avantageusement parmi les ampoules de verre, les flacons de verre ou les seringues pré-remplies.

13. Récipient de conditionnement contenant la composition selon l'une quelconque des revendications 1 à 9.

## Claims

1. Vinflunine pharmaceutical composition, **characterized in that** it is in the form of a stable and sterile aqueous solution of a watersoluble vinflunine salt at a pH of between 3 and 4 and **in that** it does not contain any preservatives agent.

2. Composition according to Claim 1, **characterized in that** the vinflunine salt is vinflunine ditartrate.

3. Composition according to Claim 2, **characterized in that** the composition consists of vinflunine ditartrate and water for an injectable preparation.

4. Composition according to Claim 1 or 2, **characterized in that** it comprises a pH buffer system in order to maintain the pH between 3 and 4.

5. Composition according to Claim 4, **characterized in that** the molarity of the pH buffer system is between 0.002 M and 0.2 M.

6. Composition according to either of Claims 4 and 5, **characterized in that** the pH buffer system consists of an acetic acid/sodium acetate buffer or a citric acid/sodium citrate buffer.

7. Composition according to any one of Claims 2 to 6, **characterized in that** the composition contains vinflunine ditartrate with a base vinflunine concentration of between 1 and 50 mg/ml, advantageously between 25 and 30 mg/ml and in particular 25 mg/ml.

8. Composition according to any one of Claims 2 to 7, **characterized in that** it corresponds to one of the following formulations: 68.35 mg of vinflunine ditartrate qs 2 ml in water or 136.70 mg of vinflunine ditartrate qs 4 ml of water or 341.75 mg of vinflunine ditartrate qs 10 ml of water, the vinflunine ditartrate corresponding, respectively, to 50 mg of base vinflunine, 100 mg of base vinflunine and 250 mg of base vinflunine.

9. Composition according to any one of the preceding claims, **characterized in that** it remains stable for at least 36 months at 5°C±3°C.

10. Use of a composition according to any one of Claims 1 to 9, for the manufacture of a medicinal product for parenteral administration, advantageously via intravenous perfusion.

11. Use according to Claim 10, **characterized in that** the medicinal product is intended for treating cancer.

12. Process for preparing a composition according to any one of Claims 1 to 9, comprising the following successive steps:
- (a) dissolution of the vinflunine salt in water for injectable preparations,
- (b) optional addition of a pH buffer,
- (c) sterilization by filtration of the bulk solution,
- (d) aseptic distribution, under a nitrogen atmosphere, of the sterile composition obtained in step (c) in the container, advantageously chosen from glass phials, glass bottles and prefilled syringes.

13. Packaging container containing the composition according to any one of Claims 1 to 9.

## Patentansprüche

1. Pharmazeutische Vinflunin-Zusammensetzung, **dadurch gekennzeichnet, dass** sie sich in Form einer sterilen und stabilen wässrigen Lösung eines wasserlöslichen Vinflunin-Salzes mit einem pH zwischen 3 und 4 inklusive darstellt und dass sie keinen Konservierungsstoff enthält.

2. Zusammensetzung nach Anspruch 1, **dadurch gekennzeichnet, dass** das Vinflunin-Salz das Vifluninditartrat ist.

3. Zusammensetzung nach Anspruch 2, **dadurch gekennzeichnet, dass** die Zusammensetzung von dem Vinflunin-Ditartrat und Wasser für injizierbares Präparat gebildet wird.

4. Zusammensetzung nach Anspruch 1 oder 2, **dadurch gekennzeichnet, dass** sie ein pH-Puffersystem umfasst, um den pH zwischen 3 und 4 zu halten.

5. Zusammensetzung nach Anspruch 4, **dadurch gekennzeichnet, dass** die Molarität des pH-Puffersystems zwischen 0,002 M und 0,2 M inklusive ist.

6. Zusammensetzung nach einem der Ansprüche 4 oder 5, **dadurch gekennzeichnet, dass** das pH-Puffersystem von einem Essigsäure/Natriumacetat-Puffer oder einem Citronensäure/Natriumcitrat-Puffer gebildet wird.

7. Zusammensetzung nach einem der Ansprüche 2 bis 6, **dadurch gekennzeichnet, dass** die Zusammensetzung Vinflunin-Ditartrat in einer Basis-Vinfluninkonzentration zwischen 1 und 50 mg/ml inklusive enthält, vorzugsweise zwischen 25 und 30 mg/ml inklusive, insbesondere 25 mg/ml.

8. Zusammensetzung nach einem der Ansprüche 2 bis 7, **dadurch gekennzeichnet, dass** sie einer der folgenden Formeln entspricht: 68,35 mg Vinflunin-Ditartrat qsp 2 ml in Wasser oder 136,70 mg Vinflunin-Ditartrat qsp 4 ml Wasser oder 341,75 mg Vinflunin-Ditartrat qsp 10 ml Wasser, wobei das Vinflunin-Ditartrat jeweils 50 mg Basisvinflunin, 100 mg Basisvinflunin und 250 mg Basisvinflunin entspricht.

9. Zusammensetzung nach einem der vorangehenden Ansprüche, **dadurch gekennzeichnet, dass** sie bei 5 °C ± 3 °C mindestens 36 Monate lang stabil bleibt.

10. Verwendung einer Zusammensetzung nach einem der Ansprüche 1 bis 9 zur Herstellung eines Arzneimittels, das zur parenteralen Verabreichung bestimmt ist, vorzugsweise auf intravenösem Weg durch Perfusion.

11. Verwendung nach Anspruch 10, **dadurch gekennzeichnet, dass** das Arzneimittel zur Behandlung des Krebses bestimmt ist.

12. Verfahren zur Zubereitung einer Zusammensetzung nach einem der Ansprüche 1 bis 9, das die folgenden aufeinanderfolgenden Schritte umfasst:
- (a) Lösung des Vinflunin-Salzes in Wasser für injizierbare Präparate,
- (b) eventuelle Zugabe eines pH-Puffers,
- (c) Sterilisation durch Filtration der losen Lösung,
- (d) aseptische Verteilung der in Schritt (c) hergestellten sterilen Lösung unter Stickstoffatmosphäre in die vorzugsweise aus Glasampullen, Glasflakons oder vorgefüllten Spritzen ausgewählte Verpackung.

13. Verpackungsbehälter, der die Zusammensetzung nach einem der Ansprüche 1 bis 9 enthält.
